# EUROPÄISCHE PATENTSCHRIFT

(11) **EP 2 095 792 B1**
(45) Veröffentlichungstag und Bekanntmachung des Hinweises auf die Patenterteilung: **26.10.2011**
(21) Anmeldenummer: 09005750.6
(22) Anmeldetag: 21.05.2002
(51) Int. Cl.: A61F 2/06, D03D 1/00, D03D 13/00

(54) **Implantat für die Chirurgie**
Surgical implant
Implant chirugical

(30) Priorität: 21.05.2001 DE 10125712
(43) Veröffentlichungstag der Anmeldung: 02.09.2009
(62) Teilanmeldung aus: 02743058.6
(73) Patentinhaber: Aesculap AG, 78532 Tuttlingen (DE)
(72) Erfinder: Bentele, Franz, 72147 Nehren (DE); Goldmann, Helmut, 78532 Tuttlingen (DE)
(74) Vertreter: Patentanwälte Ruff, Wilhelm, Beier, Dauster & Partner

(56) Entgegenhaltungen:
- EP-A- 0 327 903
- WO-A-00/51524
- WO-A-99/11198
- WO-A-99/40875
- US-A- 5 496 364
- US-A- 6 136 022

## Beschreibung

Die Erfindung betrifft ein Implantat für die Chirurgie, ein Verfahren zu seiner Herstellung sowie seine Verwendung.

Bei der medizinischen Behandlung von Menschen oder Tieren ist es bei chirurgischen Eingriffen häufig erforderlich, erkrankte oder verletzte Körperteile durch Implantate zu unterstützen, sie teilweise oder sogar ganz durch Prothesen zu ersetzen. Als Beispiele solcher Implantate sind Gefässprothesen zu nennen, die geschädigte Teile von Blutgefässen ersetzen sollen. Bekannt sind Implantate in gewirkter, gestrickter oder gewebter Konstruktion. Ferner sind Gefässprothesen mit Oberflächenstrukturierungen bekannt, wie beispielsweise mit Plissierungen oder Velour.

Bei den bekannten Implantaten zeigen sich Probleme beim Einheilungsverhalten, da der Implantatfremdkörper nur zögerlich von körpereigenen Zellen besiedelt wird. Auch zeigen sich Schwierigkeiten beim Einnähen der Prothese und der Verbindung mit dem Körpergewebe des Patienten. Bedingt durch die Porosität bekannter textiler Implantate kann es zu Undichtigkeiten mit unerwünschten Blutverlusten kommen. Dies kann zu erhöhten intraoperativen Komplikationen, verlängerten Zeiten der Unterbrechung der Blutversorgung der distalen Gewebe und Organe oder zu Wanddurchblutungen führen, mit den Risiken weiterer postoperativer Komplikationen, Einheilungsstörungen oder Folgeerkrankungen, die sich hieraus ergeben.

Aus der US 5,496,364 geht eine Gefäßprothese mit Leinwandbindung hervor, die sowohl in Kett- als auch in Schussrichtung verlaufende Flottungen aufweist.

Es stellt sich daher die Aufgabe, ein Implantat für die Chirurgie zur Verfügung zu stellen, das die Probleme von Implantaten aus dem Stand der Technik überwindet, das schnell und komplikationslos im Patientenkörper einheilt, das einfach und nach herkömmlichen Methoden kostengünstig herzustellen ist sowie in der Praxis der Chirurgie gut anwendbar ist.

Diese Aufgabe wird gelöst durch ein Implantat gemäß Anspruch 1 für die Chirurgie gebildet aus bioverträglichem Fasermaterial als gewebtes textiles Flächengebilde in Form einer Gefässprothese, dadurch gekennzeichnet, dass das Gewebe so ausgebildet ist, dass es eine Durchlässigkeit für mit Gerinnungshemmern versetztes Blut aufweist, die so gering ist, dass das Blut das textile Flächengebilde bei Implantation imprägniert und durch Gerinnung abdichtet.

Überraschenderweise wurde gefunden, dass ein Implant gemäss der vorliegenden Erfindung ein besonders rasches und komplikationsloses Einwachsverhalten zeigt. Es bilden sich keine Ansammlungen von Blutgerinnseln oder Blutkuchen, die die Ausbildung der Neointima oder der Gefässkapsel bleibend stören können.

Als Materialien zur Ausbildung eines solchen Implantats können bioverträgliche natürliche Fasern, synthetische Fasern, Mischfasern, Verbundfasern oder Mischungen daraus verwendet werden. Als Beispiel für bevorzugte Implantatmaterialien ist Polyethylenterephthalat (z. B. unter der Marke Dacron im Handel erhältlich) zu nennen.

Das erfindungsgemässe Implantat kann eine Wasserdurchlässigkeit im Bereich von 100 bis 300 ml/cm2.min, insbesondere 150 bis 250 ml/cm2.min aufweisen. Diese Wasserdurchlässigkeitswerte wurden nach der Bestimmungsmethode von Wesolowski bei 120 mmHg gemessen. Auf diese Weise zeigt sich das Implantat nach kurzer Zeit blutdicht und das Risiko von Sickerblutungen ist gering.

Mit Vorteil kann das Implantat so dünn ausgebildet sein, dass ein Einwachsen von Blutgefässen möglich ist. Es kann ferner so dünn ausgebildet sein, dass ein Durchtritt von Nährstoffen zur Versorgung einer sich ausbildenden Neointima möglich ist. Es ist Diffusion von Gewebeflüssigkeit intrazellular und durch Kapillaren möglich. Während der Ausbildung einer Neointima ist eine solche Diffusion von aussen zur Nährstoffversorgung vorteilhaft. Später erfolgt die Versorgung mittels neuer Gefässdurchwachsungen.

In einer Ausführungsform der Erfindung kann das Gewebe in einer Dicke von 0,1 bis 0,35 mm, insbesondere 0,15 bis 0,25 mm, bevorzugt 0,2 mm ausgebildet sein. Die Dickenbestimmung erfolgt nach DIN 863.

In einer besonderen Ausführungsform kann im Implantat das Gewebe mit flach nebeneinanderliegenden Fäden, ohne starken Anschlag ausgebildet sein. Bevorzugt kann das Gewebe mit Fäden der Garnstärke 10 bis 200 dtex, 10 bis 200 Filamenten pro Faden und einer Verzwirnung des Garns von 50 bis 500 Umdrehungen/m ausgebildet sein. Ausserdem kann das Gewebe mit 30 bis 120 Kettfäden und 10 bis 70 Schussfäden pro Zentimeter, insbesondere 40 bis 80 Kettfäden und 20 bis 50 Schussfäden ausgebildet sein. Erfindungsgemäss bevorzugt kann das Gewebe mit einem Rapport von 8 x 8 ausgebildet sein.

Im Gewebe des Implantats kann der Abstand der Fäden zueinander der Fadenbreite entsprechen, so dass die Fäden dicht an dicht nebeneinander liegen. Der geringe Abstand zwischen Einzelfasern ist wichtig für reizloses und schnelles Einwachsen des Implantats. Wenn sich sehr grosse Zellen zwischen Implantatfasern einlagern können, wird ein Einwachsen der gewünschten Neointima behindert. Die Einzelfasern das Fadens können im Gewebe auch flach nebeneinander liegen, so dass der Fadenquerschnitt nicht rund, sondern oval ist. Typischerweise können in einem flach liegenden Faden im Gewebe etwa 20 Einzelfasern nebeneinander liegen. Insbesondere an der Oberfläche des Implantatgewebes können flache Faserbündel mit im wesentlichen parallel verlaufenden Fasern liegen.

In einer bevorzugten Ausführungsform kann im Implantat das Gewebe im wesentlichen in Leinwandbindung ausgebildet sein, insbesondere kann es ein Grundgerüst mit Leinwandbindung besitzen.

Als Kettfäden sind nur glatte Fäden verwendet.

Als Schussfäden sind nur glatte Fäden verwendet. Mit Vorteil kann sich das Implantat dadurch auszeichnen, dass glatte Fäden leicht gefacht sind.

Flottierende Fäden sind eingebracht. Ein solcher Flottungsfaden ist vorzugsweise zusätzlich zu und parallel neben einem Kettfaden in der Leinwandbindung vorhanden. Der zusätzliche Flottungsfaden läuft dort, wo er in Leinwandbindung liegt, vorzugsweise zusammen mit dem parallelen Kettfaden und trifft sich nach der Flottung wieder mit dem Kettfaden. Bevorzugt können die Flottungsfäden in der Gewebefläche flach liegen und keine Schlaufen bilden. Mit besonderem Vorteil können die flottierenden Fäden texturiert sein. In einer bevorzugten Ausführungsform der Erfindung kann ein flottierender texturierter Faden in Nachbarschaft zu zwei in gleicher Richtung verlaufenden glatten Fäden des Gewebes in der Gewebebindung liegen. Eine Flottung in Kettrichtung kann sich bei einem röhrenförmig ausgebildeten Implantat günstig auf das Einwachsverhalten auswirken.

In einer besonderen Ausführungsform kann ein Gewebe nur aus glatten Fäden mit einer Flottung aus texturierten Fäden ausgebildet sein.

Die Flottungsfäden verlaufen nur in Kettrichtung.

Erfindungsgemäss bevorzugt kann die Flottung über mehr als 2 Fäden verlaufen, insbesondere über 3 bis 10 Fäden, bevorzugt über 4 bis 6 Fäden verlaufen. Vorteilhaft kann die Flottung 4 bis 6 über 1, bevorzugt 5 über 1 betragen. In einer bevorzugten Ausführungsform der Erfindung kann bei einem Rapport von 8 x 8 eine Flottung über 5 unter 1 über 1 unter 1 eingebracht sein. Bei der Flottung ist eine ungerade Zahl bevorzugt, da sonst der Flottungsfaden nicht mehr parallel zu einem benachbarten Gewebefaden der Leinwandbindung liegt.

Gemäss der Erfindung kann das Verhältnis von Flottungsfäden zu Kettfäden zwischen 1 : 20 bis 1 : 1, insbesondere zwischen 1 : 10 bis 1 : 2, bevorzugt bei 1 : 3 liegen. Mit anderen Worten können Flottungsfäden in einem Anteil von etwa 5 bis 50 %, insbesondere von 9 bis 33 %, bevorzugt von 25 % in Bezug auf Kettfäden im Gewebe vorhanden sein. Bevorzugt können die Flottungen von benachbarten Flottungsfäden in Kettrichtung versetzt sein, wobei sich vorzugsweise mindestens ein Teil der Flottungen überschneidet.

Es können in Schussrichtung maximal zwei Überlegungen von Kettfäden nebeneinander liegen.

Das erfindungsgemässe Implantat kann sich dadurch auszeichnen, dass die Flottungsfäden nur auf einer Oberfläche des Implantats flottieren, die vorzugsweise bei einer Gefässprothese die äussere Oberfläche ist. In Weiterbildung kann eine Flottung nur eine geringe Polhöhe aufweisen, insbesondere nur eine solche, die durch die Texturierung der Fäden bedingt ist.

Mit Vorteil kann das Gewebe durch thermische Behandlung geschrumpft sein. Insbesondere können texturierte Fäden im Gewebe durch die Schrumpfung geöffnet sein. Ausserdem kann durch eine Schrumpfung der Fäden die Polhöhe des Implantatgewebes beeinflusst sein.

Erfindungsgemäss kann das Implantat insbesondere mindestens auf einer Seite des Gewebes eine Struktur aufweisen, die ein Einwachsen einer Neointima begünstigt. Dies ist vorzugsweise eine im wesentlichen reine Leinwandbindung mit überwiegend glatten Fäden und einem geringen Anteil an offenliegenden texturierten Fäden. Mesothelzellen können ausgehend von Gefässstümpfen eine neue dünne und glatte Neointima bilden.

In Weiterbildung kann das Implantat mindestens auf einer Seite, vorzugsweise der anderen Seite des Gewebes eine Struktur aufweisen, die die Ausbildung einer dünnen Bindegewebskapsel begünstigt. Diese Seite weist im Vergleich zur anderen Seite die Flottungen auf, die insbesondere aus texturierten Fäden bestehen. Fibroblasten wachsen ausgehend von umliegendem Gewebe ein und bilden das Kollagen für eine äussere und von Blutgerinnseln freie Verkapselung.

Erfindungsgemäss kann das Gewebe als Einfachvelour ausgebildet sein. Mit Vorteil kann beim Implantat eine Velourstruktur nur auf der Gefässaussenseite ausgebildet sein. Ferner kann die Gefässinnenseite eine Struktur aufweisen, die ein Einwachsen einer Neointima begünstigt.

Eine solche Schicht aus Mesothelzellen kann sich besonders auf einer durch die Gewebekonstruktion glatten Wandung ausbilden. Bevorzugt kann das erfindungsgemässe Implantat auf einer Seite, insbesondere auf der bei einer Gefässprothese innen liegenden Seite, an weniger als 30 %, insbesondere an weniger als 20 % der Kreuzungsstellen von Kette und Schuss texturierte Fäden an der Oberfläche liegend aufweisen. Durch parallele Legung von texturierten zusätzlichen Flottungsfäden mit gleichlaufenden glatten Fäden des Grundgewebes wird der Anteil an texturierten Fäden durch teilweises Überdecken des texturierten Fadens durch den glatten Faden noch verringert. Ausserdem kann das erfindungsgemässe Implantat, bevorzugt auf der bei einer Gefässprothese aussen liegenden Seite, an weniger als der Hälfte, insbesondere an weniger als einem Drittel der Kreuzungsstellen von Kette und Schuss texturierte Fäden an der Oberfläche liegend aufweisen. Auf diese Weise kann der Flottungsfaden auf einer vorzugsweise nur eine Leinwandbindung zeigenden Seite des Implantatgewebes von normalen glatten parallelen Fäden des Leinwandgrundgewebes mindestens teilweise verdeckt sein, und auf der anderen Seite auf einem normalen Faden des Leinwandgrundgewebes verlaufen und auf der Gewebeoberfläche im wesentlichen freiliegen.

Durch die vorzugsweise Verwendung der Leinwandbindung bei der Innenseite einer Gefässprothese wird eine glatte innere Oberfläche erzeugt. Außen dagegen wird durch die erfindungsgemäße textile Struktur unter stärkerer Heranziehung der texturierten Garne eine voluminöse Struktur, verglichen mit der Innenseite, hergestellt. Durch diese stärker strukturierte, äußere Oberfläche wird ein Dichtegradient (Posositätsgradient) hin zur inneren Oberfläche der Gefässprothese erhalten, womit ein verbessertes Einwachsen der Blutgefässe bei gleichzeitig begünstigter Ausbildung der Neointima erreicht wird.

Falls in besonderen Fällen erwünscht, kann auf das Implantat gemäss der Erfindung eine schnell resorbierbare Beschichtung aus z. B. Gelatine oder anderen geeigneten natürlichen oder synthetischen Materialien aufgebracht sein. Dies ist aber in der Regel nicht erforderlich, da das Gewebe nach erstem Durchdringen mit Blut selbst abdichtet.

Es wird auch ein Verfahren zur Herstellung eines Implantats für die Chirurgie aus bioverträglichem Fasermaterial als gewebtes textiles Flächengebilde, insbesondere in Form einer Gefässprothese, das dadurch gekennzeichnet ist, dass durch Weben eine Struktur so ausgebildet wird, dass sie eine Durchlässigkeit für mit Gerinnungshemmern versetztes Blut aufweist, die so gering ist, dass das Blut das textile Flächengebilde imprägniert und durch Gerinnung abdichtet, beschrieben.

Aus einem gemäss der Erfindung hergestellten textilen Flächengebilde kann nach den Fachleuten bekannten textilen Techniken in Implantat in gewünschter Form hergestellt werden. Zur Verwendung als Gefässprothese kann das Implantat schlauchförmig mit geeignetem Lumen ausgebildet werden.

Ferner können dem erfindungsgemässen Implantat mit Vorteil ein oder mehrere medizinisch wirksame Substanzen zugesetzt sein. Solche Wirksubstanzen können beispielsweise Medikamente, Antibiotika, Antiseptika, Gerinnungsfaktoren, Wachstumsfaktoren und dergleichen sein.

Das gemäss der Erfindung hergestellte Implantat kann zur Bereitstellung für medizinische Verwendung auf an sich bekannte Weise vorbereitet werden. Insbesondere kann das erfindungsgemässe Material in geeigneter Weise sterilisiert werden. Ein zweckmässiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfasst die Behandlung mit Gammastrahlung. Ein anderes Verfahren zur Sterilisierung des erfindungsgemässen Implantatmaterials für medizinische Zwecke umfasst die Verwendung von Ethylenoxid. Mit Vorteil kann das hergestellte medizinische Implantat in zweckmässiger Grösse zugeschnitten gebrauchsfertig in geeigneter Weise verpackt vorliegen.

Ferner betrifft die Erfindung die Verwendung eines Implantats in der Chirurgie, insbesondere in Form einer Gefässprothese zur Behandlung von Defekten an Blutgefässen in der Humanmedizin und in der Veterinärmedizin.

In der tierexperimentellen Prüfung (Hund) einer gemäss der Erfindung hergestellten Gefässprothese zeigte sich diese als blutdicht. Zur Implantation der erfindungsgemässen Gefässprothese als infrarenaler Aortenersatz wird nach Freipräparation des betreffenden Aortenabschnitts proximal und distal das Gefäss abgeklemmt und Blutgerinnungshemmer Heparin eingespritzt. Nach Resektion des präparierten Abschnitts wird die proximale Anastomose angelegt. Dann wird das distale Prothesenende ebenfalls mit einer Klemme verschlossen und die proximale Klemme kurzzeitlich geöffnet, so dass heparinisiertes Blut in das Implantat eindringt und darin enthaltene Luft verdrängt. Nun wird die proximale Klemme für 15 Sekunden. geschlossen und das durch das Implantat ausgetretene Blut abgetupft. Durch erneutes Öffnen des abgeklemmten proximalen Endes des Prothesenimplantats erfolgt ein zweiter Druckstoss von Blut, wobei schon wesentlich weniger Blut durch die Prothese hindurchtritt. Das in das Gewebe des Implantats eindringende Blut gerinnt in der Gefässwandung und führt so zu einer Abdichtung der Gefässprothese. Es sind etwa zwei bis drei Blutdurchströmungen erforderlich, bis die Gefässprothese abgedichtet ist. Danach wird auch das distale Ende des Implantats vernäht.

Nach der Implantierung bildet sich schnell eine dünne Fibrinschicht in der Gefässprothesenwandung. Diese wird problemlos physiologisch abgebaut, während sich eine Neointima und eine äussere Kollagenschicht aufbauen. Überraschenderweise zeigt sich schon nach drei Monaten eine gleichmässige Ausbildung einer hellen Neointima. Drei Monate nach der Implantation zeigt sich auch eine dünne durchsichtige Bindegewebskapsel. Nach sechs Monaten ist das Implantat komplikationslos und ohne Entzündungen eingeheilt.

Beim erfindungsgemässen Implantat ist nicht nur die Porosität, sondern auch die Größe der Poren und deren Porengrößenverteilung, erzeugt durch die unterschiedliche Dichte der Fadenlegung mit unterschiedlichem Luftgehalt in der gewebten Wandung von Bedeutung. Es treten nur geringe Blutungen in der Wandung auf, daher gibt es wenig Blutgerinnsel, deren Anwesenheit Probleme bereiten kann und die abgebaut werden müssen.

Weitere Merkmale und Einzelheiten der Erfindung ergeben sich aus der nachfolgenden Beschreibung einer bevorzugten Ausführungsform, die als Beispiel mit Bezug zu einer begleitenden Figur angegeben ist. Dabei können die einzelnen Merkmale jeweils für sich alleine oder zu mehreren in Kombination miteinander verwirklicht sein. Das Beispiel dient lediglich der Erläuterung der vorliegenden Erfindung, die in keiner Weise darauf beschränkt sein soll.

### Beispiel

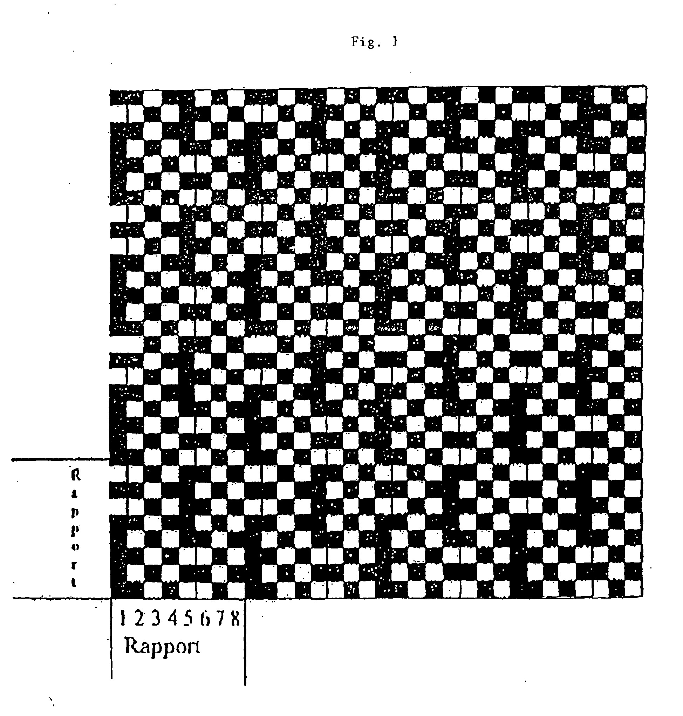

Eine Gefässprothese wird aus bioverträglichem Synthesefasermaterial (Polyethylenterephthalat) durch Weben eines Grundgewebes in Leinwandbindung mit einer Flottung in Kettrichtung ausgebildet. Das Bindungsbild ist in Fig. 1 dargestellt.

Als Kettfäden sind glatte (100 f 80 Z 240) und texturierte (100 f 80 Z 140) Fäden verwendet, wobei jeder dritte Kettfaden des Leinwandgrundgewebes texturiert ist. Der Schuss erfolgt nur mit glatten Fäden (100 f 80). Der Flottungsfaden ist texturiert (50 f 40/2).

Alle Kett- und Schussfäden sind in Leinwandbindung verlegt. Das Gewebe ist mit 31 Schussfäden und 68 Kettfäden pro Zentimeter ausgebildet. Der Flottungsfaden wird, bei einem Rapport von 8 x 8, zusätzlich zum 1. und 5. Kettfaden im Rapport eingebracht. Die Flottung ist über drei Kettfäden versetzt. In Schussrichtung überspringt der Flottungsfaden jeweils fünf Schussfäden, so dass die Flottung nur auf einer Seite des Gewebes erscheint.

Auf diese Weise sind in Kettrichtung pro Rapport vier texturierte Fäden vorhanden, davon zwei im Leinwandgrundgewebe und zwei als zusätzliche Flottungsfäden. Die zusätzlichen texturierten Flottungsfäden liegen jeweils zwischen zwei glatten Fäden. Da der Flottungsfaden texturiert ist, bildet er auf der einen Oberfläche des Gewebes eine Struktur aus. Auf der anderen Seite des Gewebes liegt der Flottungsfaden neben dem Kettfaden in der Leinwandbindung und trägt auf dieser Seite nicht zu einer Strukturierung bei.

Die Wasserdurchlässigkeit dieses Gewebes liegt im Bereich von 200 + 50 ml/cm2/min, wie nach der Bestimmungsmethode von Wesolowski bei 120 mmHg gemessen.

Die Wanddicke der unbeschichteten Prothese beträgt 0,15 bis 0,25 mm. Die Messung der Wanddicke erfolgt nach DIN 863 mittels einer Messschraube, deren Messspindel über eine Kupplung angetrieben ist. Die auf die Messfläche wirkende Kraft beträgt 5 bis 10 N. Die Messung wird an einer aufgeschnittenen Gefässprothese vorgenommen, wobei eine eventuelle Plissierung geglättet ist.

Die Wanddicke des Gewebes ist für Gefässprothesen im Bereich von 6 mm bis 38 mm Aussendurchmesser mit einer Abweichung von + 0,01 mm annähernd gleich.

## Patentansprüche

1. Implantat für die Chirurgie gebildet aus bioverträglichem Fasermaterial als gewebtes textiles Flächengebilde in Form einer Gefässprothese, wobei das Gewebe so ausgebildet ist, dass es eine Durchlässigkeit für mit Gerinnungshemmern versetztes Blut aufweist, die so gering ist, dass das Blut das textile Flächengebilde bei Implantation imprägniert und durch Gerinnung abdichtet, wobei als Kettfäden nur glatte Fäden und als Schussfäden nur glatte Fäden verwendet sind und dass flottierende Fäden eingebracht sind **dadurch** gekennzeïchnet, dass die Flottungsfäden nur in Kettrichtung verlaufen.

2. Implantat nach Anspruch 1, **dadurch** gekennnzeichnet, dass das Gewebe eine Wasserdurchlässigkeit im Bereich von 100 bis 300 ml/cm² min, insbesondere von 150 bis 250 ml/cm² min nach der Bestimmungsmethode nach Wesolowski bei 120 mmHg aufweist.

3. Implantat nach Anspruch 1 oder 2, **dadurch gekennzeichnet, dass** das Gewebe in einer Dicke von 0,1 bis 0,35 mm, insbesondere 0,15 bis 0,25 mm, bevorzugt 0,2 mm ausgebildet ist.

4. Implantat nach einem der vorhergehenden Ansprüche, **dadurch gekennzeichnet, dass** das Gewebe mit Fäden der Garnstärke 10 bis 200 dtex, 10 bis 200 Filamenten pro Faden und einer Verzwirnung des Garns von 50 bis 500 Umdrehungen/m ausgebildet ist.

5. Implantat nach einem der vorhergehenden Ansprüche, **dadurch gekennzeichnet, dass** das Gewebe mit 30 bis 120 Kettfäden und 10 bis 70 Schussfäden pro Zentimeter, insbesondere 40 bis 80 Kettfäden und 20 bis 50 Schussfäden ausgebildet ist.

6. Implantat nach einem der vorhergehenden Ansprüche, **dadurch gekennzeichnet, dass** das Gewebe im wesentlichen in Leinwandbindung ausgebildet ist, insbesondere ein Grundgerüst mit Leinwandbindung besitzt.

7. Implantat nach einem der vorhergehenden Ansprüche, **dadurch gekennzeichnet, dass** die Flottungsfäden in der Gewebefläche flach liegen und keine Schlaufen bilden.

8. Implantat nach einem der vorhergehenden Ansprüche, **dadurch gekennzeichnet, dass** die Flottung über mehr als 2 Fäden verläuft, insbesondere über 3 bis 10 Fäden, bevorzugt über 4 bis 6 Fäden verläuft.

9. Implantat nach einem der vorhergehenden Ansprüche, **dadurch gekennzeichnet, dass** die Flottung 4 bis 6 über 1, bevorzugt 5 über 1 beträgt, wobei zwischen zwei Flottierungen vorzugsweise jeweils eine Leinwandbindung vorgesehen ist.

10. Implantat nach einem der vorhergehenden Ansprüche, **dadurch gekennzeichnet, dass** das Verhältnis von Flottungskettfäden zu in Leinwandbindung liegenden Kettfäden zwischen 1 : 20 bis 1 : 1, insbesondere zwischen 1 : 10 bis 1 : 2, bevorzugt bei 1 : 3 liegt.

11. Implantat nach einem der vorhergehenden Ansprüche, **dadurch gekennzeichnet, dass** die Flottungsfäden nur auf einer Oberfläche des Implantats flottieren, die vorzugsweise bei einer Gefäßprothese die äußere Oberfläche ist.

12. Implantat nach einem der vorhergehenden Ansprüche mit einer inneren und einer äußeren Oberfläche, **dadurch gekennzeichnet, dass** die innere Oberfläche im wesentlichen glatt ausgebildet ist, wobei die äußere Oberfläche vorzugsweise strukturiert ist.

13. Implantat nach einem der Ansprüche 1 bis 12 zur Verwendung in der Chirurgie, insbesondere in Form einer Gefäßprothese zur Behandlung von Defekten an Blutgefäßen in der Humanmedizin und in der Veterinärmedizin.

## Claims

1. Surgical implant made from biocompatible fiber material as a woven textile fabric in the form of a vascular prosthesis, the woven fabric being so configured that its permeability to blood, to which anticoagulants have been added, is so low that the blood impregnates the textile fabric upon implantation and seals it off by coagulating, only flat threads being used as warp threads and only flat threads as weft threads and floating threads being included, **characterized in that** the floating threads extend only in the warp direction.

2. Implant according to Claim 1, **characterized in that** the woven fabric has a water permeability in the range of from 100 to 300 ml/cm².min, in particular of from 150 to 250 ml/cm².min using the Wesolowski method of determination at 120 mmHg.

3. Implant according to Claim 1 or 2, **characterized in that** the woven fabric is configured in a thickness of from 0.1 to 0.35 mm, in particular of from 0.15 to 0.25 mm, preferably 0.2 mm.

4. Implant according to one of the preceding claims, **characterized in that** the woven fabric is configured with threads of yarn size 10 to 200 dtex, 10 to 200 filaments per thread, and a twisting of the yarn of 50 to 500 turns/m.

5. Implant according to one of the preceding claims, **characterized in that** the woven fabric is configured with 30 to 120 warp threads and 10 to 70 weft threads per centimeter, in particular 40 to 80 warp threads and 20 to 50 weft threads.

6. Implant according to one of the preceding claims, **characterized in that** the woven fabric is configured essentially in a plain weave, in particular having a base structure with a plain weave.

7. Implant according to one of the preceding claims, **characterized in that** the floating threads lie flat in the woven fabric surface and do not form loops.

8. Implant according to one of the preceding claims, **characterized in that** the floating extends over more than 2 threads, in particular over 3 to 10 threads, preferably over 4 to 6 threads.

9. Implant according to one of the preceding claims, **characterized in that** the floating is 4 to 6 over 1, preferably 5 over 1, a plain weave being preferably provided in each case between two floats.

10. Implant according to one of the preceding claims, **characterized in that** the ratio of floating warp threads to warp threads lying in the plain weave is between 1:20 to 1:1, in particular between 1:10 to 1:2, preferably 1:3.

11. Implant according to one of the preceding claims, **characterized in that** the floating threads float only on one surface of the implant, which is preferably the outer surface in the case of a vascular prosthesis.

12. Implant according to one of the preceding claims with an inner surface and an outer surface, **characterized in that** the inner surface is substantially smooth, and the outer surface is preferably structured.

13. Implant according to one of Claims 1 to 12 for use in surgery, in particular in the form of a vascular prosthesis for treating blood vessel defects in human medicine and veterinary medicine.

## Revendications

1. Implant pour chirurgie, formé de matériau fibreux biocompatible se présentant en tant que structure textile tissée sous forme d'une prothèse vasculaire, le tissu étant réalisé de manière à présenter une perméabilité au sang transformé par des inhibiteurs de coagulation assez faible pour que le sang imprègne la structure textile lors de l'implantation et assure l'étanchéité par la coagulation, seulement des fils lisses étant employés comme fils de chaîne et seulement des fils lisses comme fils de trame, et des fils flottants y étant intégrés, **caractérisé en ce que** les fils flottants ne s'étendent que dans le sens de la chaîne.

2. Implant selon la revendication 1, **caractérisé en ce que** le tissu présente une perméabilité à l'eau de l'ordre de 100 à 300 ml/cm² par min, notamment de 150 à 250 ml/cm² par min suivant le procédé de détermination de Wesolowski à 120 mm/g.

3. Implant selon la revendication 1 ou 2, **caractérisé en ce que** le tissu est réalisé en une épaisseur de 0,1 à 0,35 mm, notamment 0,15 à 0,25 mm, préférentiellement 0,2 mm.

4. Implant selon une des revendications précédentes, **caractérisé en ce que** le tissu est réalisé avec des fils d'une épaisseur de 10 à 200 dtex, 10 à 200 filaments par fil et un retordage du fil de 50 à 500 rotations/m.

5. Implant selon une des revendications précédentes, **caractérisé en ce que** le tissu est réalisé avec 30 à 120 fils de chaîne et 10 à 70 fils de trame par centimètre, notamment 40 à 80 fils de chaîne et 20 à 50 fils de trame.

6. Implant selon une des revendications précédentes, **caractérisé en ce que** le tissu est réalisé sensiblement en armure toile et possède notamment une structure de base avec armure toile.

7. Implant selon une des revendications précédentes, **caractérisé en ce que** les fils flottants sont à plat dans la surface du tissu et ne forment pas de boucles.

8. Implant selon une des revendications précédentes, **caractérisé en ce que** le flottage passe sur plus de 2 fils, notamment 3 à 10 fils, préférentiellement 4 à 6 fils.

9. Implant selon une des revendications précédentes, **caractérisé en ce que** le flottage est de 4 à 6 sur 1, préférentiellement 5 sur 1, une armure toile étant prévue de préférence respectivement entre deux flottages.

10. Implant selon une des revendications précédentes, **caractérisé en ce que** le rapport entre les fils de chaîne flottants et les fils de chaîne se trouvant dans l'armure toile va de 1/20 à 1/1, notamment de 1/10 à 1/2 et est préférentiellement de 1/3.

11. Implant selon une des revendications précédentes, **caractérisé en ce que** les fils flottants flottent seulement sur une surface de l'implant qui est préférentiellement la surface extérieure dans le cas d'une prothèse vasculaire.

12. Implant selon une des revendications précédentes, comprenant une surface intérieure et une surface extérieure, **caractérisé en ce que** la surface intérieure a une conformation sensiblement lisse, la surface extérieure étant préférentiellement structurée.

13. Implant selon une des revendications 1 à 12, destiné à un usage en chirurgie, notamment sous forme d'une prothèse vasculaire pour le traitement des défauts des vaisseaux sanguins en médicine humaine et en médecine vétérinaire.
